# NEUE EUROPÄISCHE PATENTSCHRIFT

(11) **EP 1 563 799 B2**
(45) Veröffentlichungstag und Bekanntmachung des Hinweises auf die Entscheidung über den Einspruch: **28.11.2012**
(45) Hinweis auf die Patenterteilung: 14.03.2007
(21) Anmeldenummer: 04003019.9
(22) Anmeldetag: 11.02.2004
(51) Int. Cl.: A61B 19/00

(54) **Verstellbare Markeranordnung**
Adjustable marker arrangement
Ensemble de repères ajustable

(43) Veröffentlichungstag der Anmeldung: 17.08.2005
(73) Patentinhaber: BrainLAB AG, 85622 Feldkirchen (DE)
(72) Erfinder: Tuma, Gregor, 81675 München (DE); Plassky, Norman, 99099 Erfurt (DE)
(74) Vertreter: Schwabe - Sandmair - Marx

(56) Entgegenhaltungen:
- WO-A-02/064042
- WO-A-2004/030558
- US-A1- 2003 225 329
- US-B1- 6 190 395
- US-B1- 6 226 548

## Beschreibung

Die vorliegende Erfindung bezieht sich auf eine verstellbare Markeranordnung und insbesondere auf ein Markersystem, welches mit einem medizinischen Navigationssystem verwendet werden kann, wobei das Markersystem einen Adapter aufweist, an welchem ein Referenzstern befestigt werden kann und welcher an einem Körper, wie zum Beispiel an einem Patienten oder an einem Instrument angebracht werden kann, um die Position des Patienten oder des Instrumentes zum Beispiel während eines medizinischen Verfahrens zu ermitteln.

Medizinische Navigationssysteme, wie zum Beispiel bildgestützte Systeme, ermöglichen es, dass zum Beispiel ein Chirurg eine Navigationsinformation durch das Registrieren von vor der Operation aufgenommenen Bildern eines Patienten zur Verfügung gestellt bekommt. Es können auch chirurgische Instrumente nach einem Kalibrierungsverfahren navigiert werden, wobei zum Beispiel die Position eines Instrumentes während eines Eingriffs relativ zu den vor dem Eingriff aufgenommenen Bildern einer Körperstruktur dargestellt werden kann.

Navigationssysteme verwenden einen Computer, mit welchem ein oder mehrere sogenannte Tracking-Sensoren oder Kameras verbunden sind, so dass die Position von Markern ermittelt werden kann, welche an einem Patienten und/oder an Instrumenten befestigt sind, wodurch die Position des Patienten und/oder der Instrumente bestimmt werden kann. Solche Marker können sowohl aktive Emitter, als auch sogenannte passive reflektierende Marker sein, welche mit Adaptern an einem Patienten und/oder an chirurgischen Instrumenten, wie zum Beispiel einem Skalpell, einer Zange, einem Mikroskop oder einem Pointer angebracht werden können. Häufig wird ein Adapter verwendet, welcher ein erstes Befestigungselement, wie zum Beispiel eine Klammer, zur Anbringung zum Beispiel an einem Instrument auf der einen Seite und ein zweites Befestigungselement zum Beispiel für einen Referenzstern mit drei oder mehr sich nach außen erstreckenden Armen an der anderen Seite aufweist, wobei an den äußeren Enden der Arme reflektierende Marker angebracht werden können oder angebracht sind.

Ein bekanntes Navigationssystem, welches die oben beschriebenen Referenzsterne verwendet, ist das von der BrainLAB AG erhältliche bildgestützte Navigationssystem VectorVision™ und ist zum Beispiel in der US 2003/0225329 A1 beschrieben.

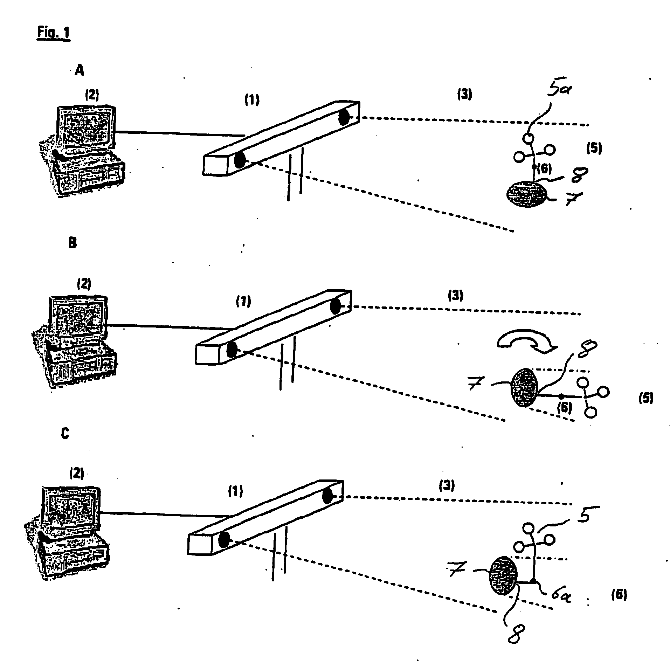

Während eines chirurgischen Eingriffes kann es vorkommen, dass zum Beispiel mehrere Referenzsterne innerhalb eines begrenzten Arbeitsbereiches eines Chirurgen angebracht sind, wobei diese Referenzsterne vorteilhaft unterschiedliche Geometrien haben, um zum Beispiel verschiedene Instrumente unterscheiden zu können. Jedoch kann die Verwendung mehrerer Referenzsterne dazu führen, dass sich diese innerhalb des Sichtfeldes einer Kamera überdecken, oder dass einzelne Marker oder Referenzsterne durch Objekte oder Personen verdeckt werden oder auch die Tätigkeit des Chirurgen behindern. Insbesondere, wenn zum Beispiel der Körper oder eine Extremität des Patienten bewegt wird, kann diese Bewegung dazu führen, dass ein an dem Körper 7 befestigter Referenzstern 5 nicht mehr von den Kameras 1 entdeckt werden kann, wie zum Beispiel in Figur 1B gezeigt. Würde der Referenzsternadapter 6 von dem Körper 7 gelöst, um den Referenzstern 5 wieder in einer anderen Position an dem Objekt anzubringen, in welcher er von den Kameras 1 erkannt werden kann, müsste der Patient neu registriert werden.

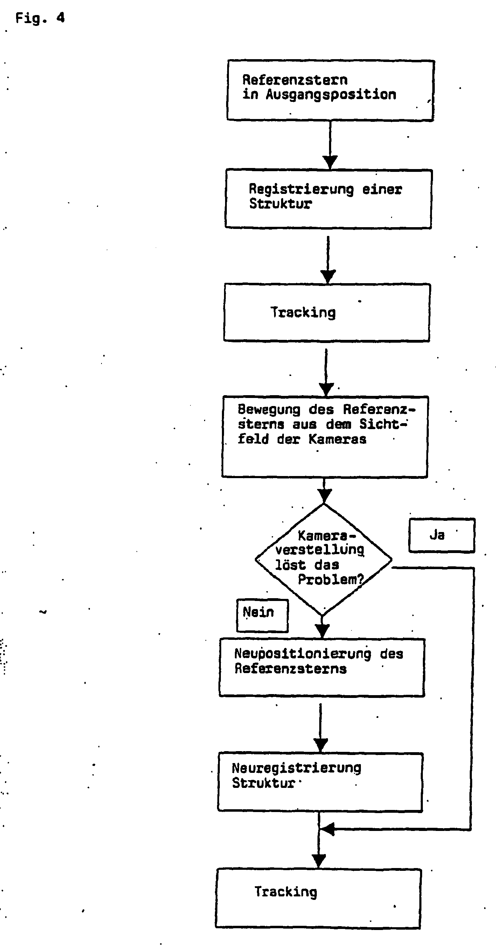

Ein Ablaufdiagramm zur Veranschaulichung der Schritte bei einer Bewegung des Referenzsterns aus dem Sichtfeld der Kameras ist in Figur 4 gezeigt.

Es ist eine Aufgabe der vorliegenden Erfindung einen Referenzsternadapter und ein Verfahren zum Neukalibrieren oder Neureferenzieren eines Instruments oder Körpers vorzuschlagen, welche bildgestützte chirurgische System effizienter und schneller machen können.

Diese Aufgabe wird durch die in den unabhängigen Ansprüchen definierten Erfindungen gelöst. Vorteilhafte Ausführungsformen ergeben sich aus den Unteransprüchen.

Gemäß einem ersten Aspekt der Erfindung weist ein Referenzsternadapter ein erstes Befestigungselement, wie zum Beispiel eine Klammer zur Befestigung des Adapters an einem Instrument oder eine Schraube zur Befestigung des Adapters an einem Körper, wie zum Beispiel einem Knochen, auf und weist ein zweites Befestigungselement auf, an welchem der Referenzstern oder auch die einzelnen Marker angebracht werden können. Erfindungsgemäß ist zwischen dem ersten Befestigungselement zur Anbringung des Adapters an einem Körper oder einem Instrument und dem zweiten Befestigungselement zur Befestigung des Referenzsternes oder der Marker mindestens ein Lager oder Gelenk vorgesehen, welches es ermöglicht, dass das zweite Befestigungselement relativ zum ersten Befestigungselement verstellt werden kann, wobei erfindungsgemäß mit dem Lager ein aktiver oder passiver Positionsdetektor verbunden ist, welcher Daten liefert, welche die Position oder Geometrie des mindestens einen Lagers beschreiben oder definieren. Durch die Verwendung eines erfindungsgemäß vorgesehenen Positionsdetektors kann zum Beispiel ein an einem Körper oder Instrument mit dem erfindungsgemäß ausgestalteten Referenzsternadapter befestigter Referenzstern automatisch oder manuell neu positioniert werden, also zum Beispiel um ein oder mehrere Gelenke gedreht und/oder entlang einer oder mehrerer vorgegebener Richtungen verschoben werden, wobei durch den mindestens einen Positionsdetektor ermittelt werden kann welche Bewegung der Referenzstern in Bezug auf seine bisherige Position ausgeführt hat. Somit ist eine neue Registrierung oder Kalibrierung nicht erforderlich, da aus der durch den mindestens einen Positionsdetektor ermittelten Lageveränderung des Referenzsternes das neue Lageverhältnis zwischen dem Referenzstern und dem mit diesem Referenzstern verbundenem Körper oder Instrument zum Beispiel durch ein Computerprogramm bestimmt werden kann und somit der Referenzstern wieder in einem definierten Lageverhältnis zu diesem Körper oder Instrument steht, wenn vorher eine Referenzierung oder Kalibrierung durchgeführt wurde. Folglich ist es nicht erforderlich, dass nach dem Verändern der Position des Referenzsternes eine neue Registrierung oder Kalibrierung durchgeführt wird, wodurch es einfacher wird während eines chirurgischen Eingriffs einen oder mehrere Referenzsterne neu zu positionieren und anschließend ohne größeren Zeitverlust weiter zu arbeiten.

Das erfindungsgemäß zwischen dem ersten und zweiten Befestigungselement vorgesehene mindestens eine Lager kann ein Loslager, wie zum Beispiel ein Querlager, Gleitlager oder Rollenlager, aber auch ein Festlager, wie zum Beispiel ein Quer- und Längslager oder ein festes Gelenk sein. Das Lager kann auch als Drehgelenk, Schubgelenk, Kurvengelenk, Schraubgelenk, Drehschubgelenk, Kugelgelenk oder Plattengelenk ausgebildet sein. Ebenso ist es möglich, dass eine Kombination unterschiedlicher Lager oder Gelenke verwendet wird, um es zu ermöglichen, dass ein an dem zweiten Befestigungselement befestigter Referenzstern in allen sechs Freiheitsgraden bewegt werden kann, wobei das erste Befestigungselement mit dem Körper oder Instrument verbunden bleiben kann, wobei durch den erfindungsgemäß bevorzugt bei jedem Lager oder Gelenk vorgesehenen mindestens einen Positionsdetektor ermittelt werden kann, welche neue Position der Referenzstern in Bezug auf die bisherige Position hat oder wie die Position des Referenzsternes verändert wurde.

Gemäß einer Ausführungsform kann ein Gelenk oder Lager des Referenzsternadapters so ausgestaltet sein, dass das Gelenk nur an definierten vorgegebenen Positionen zum Beispiel durch eine Verzahnung oder Verrastung festgestellt oder arretiert werden kann, wobei zum Beispiel ein Rastmechanismus an einem Gelenk oder Lager vorgesehen ist, welcher es zwar ermöglicht, dass das Gelenk kontinuierlich bewegt werden kann, wobei jedoch ein stabiler Zustand nur an bestimmten vorgegebenen Rastpositionen vorliegt.

Vorzugsweise können an den vorgegebenen definierten Positionen des Lagers oder Gelenks Markierungen vorgesehen sein, welche als Positionsdetektoren verwendet werden, so dass ein Benutzer des erfindungsgemäßen Referenzsternadapters an der Markierung des Lagers oder Gelenkes ablesen kann in welcher Position sich das Gelenk oder Lager momentan befindet. Bei dem kontinuierlich verstellbaren Lager oder Gelenk ist eine kontinuierliche Anzeige vorgesehen, welche beispielsweise bei einem Drehgelenk den Öffnungswinkel des Gelenks anzeigt, so dass ein Benutzer zum Beispiel nach dem Arretieren des Gelenks ablesen kann wie der momentane Zustand des Gelenks ist bzw. um wie viel die Lage des Gelenkes oder des damit verbundenen Referenzsternes verändert wurde. Ebenso ist es möglich, dass Positions- oder Lagesensoren an einem oder mehreren Gelenken oder Lagern vorgesehen sind, welche die Bewegung eines Gelenks oder Lagers messen oder ermitteln können. Beispielsweise können Winkelsensoren oder Linearsensoren verwendet werden, welche eine Rotation, wie zum Beispiel einen Öffnungswinkel eines Gelenks oder eine Translation, wie zum Beispiel die Verschiebung einer gelagerten Achse quantitativ ermitteln können, wobei die ermittelten Positionsgrößen zum Beispiel angezeigt und manuell eingegeben und/oder automatisch an das Navigationssystem übertragen werden können. Beispielweise können die einzelnen Lagesensoren über Kabel, Funk, wie zum Beispiel Bluetooth, oder eine Infrarotschnittstelle mit dem Navigationssystem verbunden werden, um die Position oder die Veränderung der Position des Referenzsternes an das Navigationssystem zu übermitteln.

Weiterhin kann der erfindungsgemäße Referenzsternadapter halbautomatisch oder automatisch ausgebildet sein, indem ein oder mehrere Motoren vorgesehen sind, mit welchen der Referenzstern bewegt werden kann, wobei erfindungsgemäß entweder an einem Motor ein Lage- oder Positionsdetektor vorgesehen ist, oder der Motor als Stellmotor betrieben wird, so dass die von dem Motor bewirkte Lageveränderung des Referenzsternes genau definiert ist und somit an das Navigationssystem übertragen werden kann.

Nach einer weiteren Ausführungsform bezieht sich die Erfindung auf ein Navigationssystem mit mindestens einem wie oben beschriebenen Referenzsternadapter und einem Referenzstern, wobei weiter eine Kamera und eine damit verbundene Recheneinheit vorgesehen sein können.

Gemäß einem weiteren Aspekt bezieht sich die Erfindung auf ein Verfahren zum Neukalibrieren und/oder Neureferenzieren eines Körpers oder Instruments mit einem daran befestigten Referenzstern, wobei die Position des Referenzsternes relativ zum Körper oder Instrument verändert wird und die Veränderung der Position des Referenzsternes detektiert wird, so dass aus der detektierten Veränderung der Position des Referenzsternes das neue Lageverhältnis zwischen dem Referenzstern und dem Körper oder Instrument ermittelt werden kann, so dass die Neukalibrierung oder Neureferenzierung einfach durchgeführt werden kann.

Vorteilhaft wird die Veränderung der Position des Referenzsternes automatisch an das Navigationssystem übertragen, so dass zum Beispiel eine kontinuierliche Neukalibrierung oder Neureferenzierung während einer Bewegung des Referenzsternes relativ zu dem Körper oder Instrument durchgeführt werden kann. Ebenso ist es möglich eine Neukalibrierung oder Neureferenzierung erst nach der Positionsveränderung unter Verwendung der detektierten Positionsveränderungsdaten durchzuführen.

Vorzugsweise ist die Geometrie oder Struktur des Adapters und/oder des mindestens einen Gelenks oder Lagers in einer Datenbank gespeichert.

Die Erfindung bezieht sich weiterhin auf ein Computerprogramm, welches, wenn es in einen Computer geladen wird oder auf einem Computer läuft, ein wie oben beschriebenes Verfahren durchführt. Des weiteren bezieht sich die Erfindung auf ein Programmspeichermedium oder ein Computerprogrammprodukt mit einem solchen Programm.

Nachfolgend wird die Erfindung anhand bevorzugter Ausführungsbeispiele beschrieben werden. Es zeigen:
- Figuren 1A bis 1C: eine Neureferenzierung eines mit einem erfindungsgemäßen Referenzsternadapter verbundenen Körpers;
- Figur 2: eine Ausführungsform eines Einstellmechanismus;
- Figur 3: ein Ablaufdiagramm bei einer erfindungsgemäßen Neuregistrierung eines Patienten; und
- Figur 4: ein Ablaufdiagramm bei einer Neuregistrierung eines Patienten nach dem Stand der Technik.

Figur 1 zeigt ein Navigationssystem, wobei Kameras 1 ein Sichtfeld 3 haben und die an einem Referenzstern 5 vorgesehenen passiven Marker 5a erfassen können. Die von den Kameras 1 erfasste Position der Marker 5a wird an eine Recheneinheit 2 weitergegeben. Der Referenzstern 5 ist mittels eines Adapters 6 mit einem Körper 7 durch ein Befestigungselement 8 verbunden, welcher auf bekannte Art referenziert wurde.

Wird der Körper 7 so gedreht wie in Figur 1B gezeigt, dass der Referenzstern 5 von dem Körper 7 verdeckt wird, so können die Kameras 1 nicht mehr die Position des Referenzsternes 5 erfassen.

Durch den erfindungsgemäßen Referenzsternadapter 6 ist es möglich, dass der Referenzstern 5 mit dem Körper 7 verbunden bleibt und durch eine manuelle oder automatische Bewegung des an dem Referenzsternadapters 6 vorgesehenen Gelenks 6a so verschoben und/oder geklappt wird, dass der Referenzstern 5 wieder in den Erfassungsbereich 3 der Kameras 1 gelangt. Da erfindungsgemäß an dem Gelenk 6a ein Sensor oder Dekoder vorgesehen ist, welcher die quantitative Bewegung des Gelenkes 6a erfassen kann und diese quantitative Information an die Recheneinheit 2 des Navigationssystems automatisch oder durch manuelle Eingabe übermittelt wird, kann der Körper 7 einfach neu referenziert werden, indem die Lageveränderung des Referenzsternes 5 ermittelt wird, wodurch das neue Lageverhältnis zwischen dem Referenzstern 5 und dem Körper 7 einfach bestimmt bzw. berechnet werden kann; wenn die Geometrie bzw. Struktur des Adapaters 6 oder des Gelenks 6a bekannt ist.

Obwohl das in Figur 1 dargestellte Funktionsprinzip der Erfindung anhand eines mit einem Körper 7 verbundenen Referenzsternes 5 beschrieben wurde, ist es ebenso möglich, dass der Referenzstern 5 zum Beispiel mit einem chirurgischen Instrument verbunden ist und dass mehr Gelenke als nur das beispielhaft dargestellte eine Gelenk 6a vorgesehen sind.

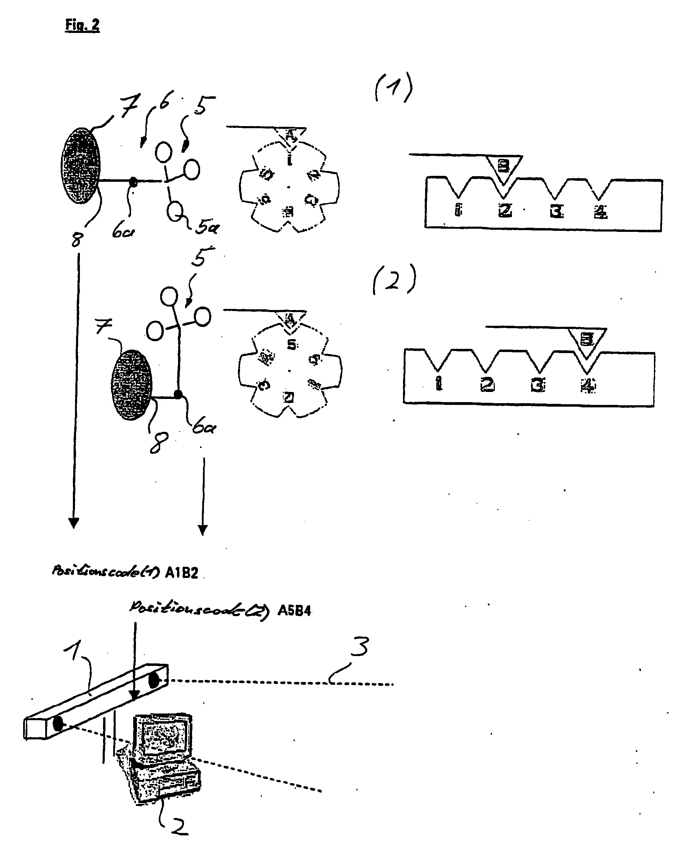

Figur 2 zeigt einen Einstellmechanismus des Referenzsternadapters 6. Ein Referenzstern 5 ist mit einem Körper oder Instrument 7 mittels des Referenzsternadapters 6 verbunden, welcher einen Verstellmechanismus 6a aufweist, der aus einem Drehgelenk A und einem Schiebelager B besteht. Das Drehgelenk A kann in sechs verschiedenen Drehpositionen 1 bis 6 verrasten, wobei in der gezeigten Ausgangsposition (1) das Drehgelenk A in der Position "1" ist. Das Schiebelager B kann in vier verschiedenen Stellungen 1 bis 4 verrastet werden, wobei in der gezeigten Ausgangsstellung (1) das Schiebelager B in der Position 2 verrastet ist.

Soll der Referenzstern 5 in eine Position gebracht werden, in welcher er nicht mehr von dem Körper 7 verdeckt wird, so kann der Referenzstern 5 manuell oder automatisch aus der in Figur 2 gezeigten Ausgangsposition (1) nach oben geklappt werden, wobei auch der Abstand des Referenzsternes 5 zum Gelenk 6a vergrößert werden kann. Nach dem Verstellen der Position des Referenzsternes 5 ist das Drehgelenk A in der in Position (2) gezeigten Drehposition "5" verrastet und das Schiebelager B ist in der gezeigten Position "4" verrastet, so dass der Positionscode (1) in der Ausgangsstellung A1B2 nach dem Verstellen des Referenzsternes 5 zu dem neuen Positionscode (2) A5B4 wird, welcher der Recheneinheit 2 des Navigationssystems 1 entweder durch einen Benutzer eingegeben werden kann, welcher diesen Positionscode zum Beispiel am Referenzsternadapter abliest, oder welcher automatisch zum Beispiel über Bluetooth an die Recheneinheit 2 des Navigationssystems übertragen werden kann. Ist dem Navigationssystem die Geometrie des Referenzsternadapters und dessen Verstellmöglichkeiten durch die Lager oder Gelenke bekannt, wie zum Beispiel die im in Figur 2 gezeigten Ausführungsbeispiel beschriebene Verstellmöglichkeit durch eine Drehung des Drehgelenkes in 60° Schritten und eine Verschiebung des Schiebelagers zu vier verschiedenen vorgegebenen Positionen, so kann daraus das neue Lageverhältnis zwischen dem verstellten Referenzstern 5 und dem Körper oder Instrument 7 berechnet werden, wodurch es einfach ist eine Neuregistrierung oder Neukalibrierung nach dem Verstellen des Referenzsternes 5 durchzuführen. Demzufolge können die aus dem Stand der Technik bekannten aufwändigen Registrierungs- und Kalibrierverfahren nach einer Neupositionierung des Referenzsternes 5 entfallen.

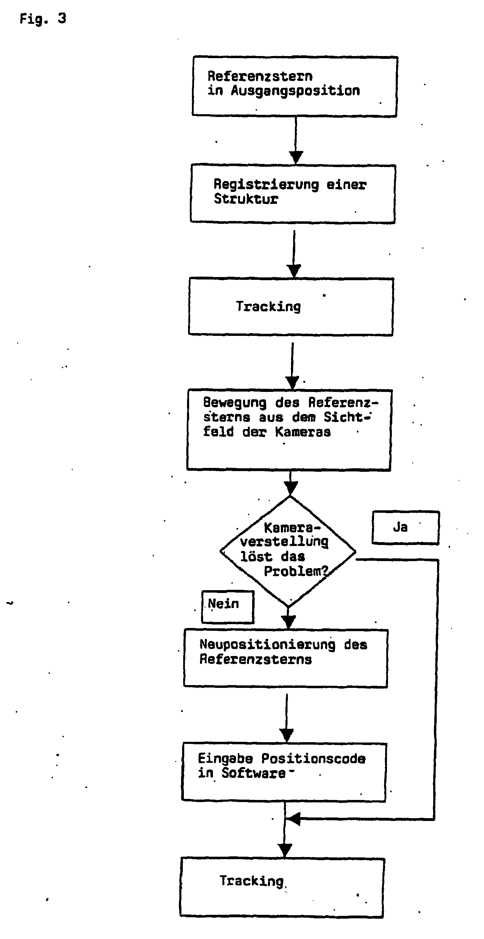

Figur 3 zeigt ein Ablaufdiagramm zum Neuregistrieren eines Patienten nach dem Verstellen der Position eines mit dem Patienten über einen erfindungsgemäßen Adapter verbundenen Referenzsternes. In der Ausgangsstellung wird der Patient, also zum Beispiel eine bestimmte Körperstruktur des Patienten, registriert und die registrierte Struktur kann getrackt werden. Wird die registrierte Struktur aus dem Sichtfeld 3 der Kameras 1 herausbewegt, so kann zunächst versucht werden, ob durch ein Verstellen der Kameras der Referenzstern wieder in das Sichtfeld gelangt. Falls dies nicht gelingt, wird der Referenzstern in eine neue Position gebracht und dem Navigationssystem bzw. der Software wird ein in Figur 2 beispielhaft gezeigter neuer Positionscode mitgeteilt, so dass daraus das neue Lageverhältnis berechnet wird und es möglich ist die dann neuregistrierte Struktur weiter zu tracken.

## Patentansprüche

1. Referenzsternadapter mit mindestens einem ersten Befestigungselement (8), um den Referenzsternadapter an einem Patienten zu befestigen und mindestens einem zweiten Befestigungselement (5), an welchem Marker (5a) angebracht sind, wobei mindestens ein kontinuierlich verstellbares Lager oder Gelenk (6) zwischen dem ersten Befestigungselement (8) und dem zweiten Befestigungselement (5) vorgesehen ist, wobei an dem mindestens einen Lager oder Gelenk (6) mindestens ein Positionsdetektor mit einer kontinuierlichen Anzeige angebracht ist, um festzustellen, in welcher Position sich das Lager oder Gelenk (6) befindet.

2. Referenzsternadapter nach Anspruch 1, wobei das Lager (6) ein Loslager oder ein Festlager ist oder das Gelenk ein Drehgelenk, Schubgelenk, Kurvengelenk, Schraubgelenk, Drehschubgelenk, Kugelgelenk oder Plattengelenk ist.

3. Referenzsternadapter nach einem der vorhergehenden Ansprüche, wobei der Positionsdetektor eine Translationsbewegung messen kann.

4. Referenzsternadapter nach einem der vorhergehenden Ansprüche, wobei der Positionsdetektor eine Rotationsbewegung messen kann.

5. Referenzsternadapter nach einem der vorhergehenden Ansprüche, wobei der mindestens eine Positionsdetektor mit einer Recheneinheit (2) verbunden ist.

6. Referenzsternadapter nach einem der vorhergehenden Ansprüche mit mindestens einem Motor, welcher die Stellung mindestens eines Lagers oder Gelenkes (6) verändern kann.

7. Navigationssystem mit einem Referenzsternadapter nach einem der vorhergehenden Ansprüche, einem Referenzstern (5), Kameras (1) und einer mit den Kameras (1) verbundenem Recheneinheit (2).

8. Verfahren zum Neukalibrieren oder Neureferenzieren eines Patienten mit einem daran befestigten Referenzstem (5), wobei mindestens ein kontinuierlich verstellbares Lager oder Gelenk (6) zwischen dem Referenzstern (5) und dem Patienten vorgesehen ist und wobei die Position des Referenzsternes (5) relativ zum Patienten verändert wird und die Veränderung der Position des Referenzsternes (5) relativ zum Patienten mittels mindestens einem Positionsdetektor mit einer kontinuierlichen Anzeige, der an dem Lager oder Gelenk (6) angebracht ist, detektiert wird, wobei kontinuierlich ermittelt werden kann, welche Bewegung der Referenzstern (5) in Bezug auf seine bisherige Position ausgeführt hat.

9. Verfahren nach dem vorhergehenden Anspruch, wobei die detektierte Veränderung der Position des Referenzsterns (5) an ein Navigationssystem übermittelt wird.

10. Verfahren nach dem vorhergehenden Anspruch, wobei in einer Software des Navigationssystems in einer Datenbank die Struktur des Referenzsternadapters und/oder des Lagers oder Gelenks (6) gespeichert ist.

11. Computerprogramm, welches, wenn es auf einem Computer läuft oder in einen Computer geladen ist, das Verfahren nach einem der drei vorhergehenden Ansprüche durchführt.

12. Programmspeichermedium oder Computerprogrammprodukt mit dem Computerprogramm nach dem vorhergehenden Anspruch.

## Claims

1. A reference star adaptor comprising at least a first fixing element (8), in order to fix the reference star adaptor to a patient, and at least a second fixing element (5) to which markers (5a) are attached, wherein at least one continuously adjustable bearing or joint (6) is provided between the first fixing element (8) and the second fixing element (5), wherein at least one position detector with a continuous display is attached to the at least one bearing or joint (6) in order to establish the position in which the bearing or joint (6) is situated.

2. The reference star adaptor according to claim 1, wherein the bearing (6) is a movable bearing or a fixed bearing or the joint is a turning joint, sliding joint, cam joint, screw joint, turning and sliding joint, ball joint or plate joint.

3. The reference star adaptor according to any one of the preceding claims, wherein the position detector can measure a translational movement.

4. The reference star adaptor according to any one of the preceding claims, wherein the position detector can measure a rotational movement.

5. The reference star adaptor according to any one of the preceding claims, wherein the at least one position detector is connected to a computational unit (2).

6. The reference star adaptor according to any one of the preceding claims, comprising at least one motor which can change the position of at least one bearing or joint (6).

7. A navigation system, comprising: a reference star adaptor according to any one of the preceding claims; a reference star (5); cameras (1); and a computational unit (2) connected to the cameras (1).

8. A method for re-calibrating or re-referencing a patient with a reference star (5) fixed to them, wherein at least one continuously adjustable bearing or joint (6) is provided between the reference star (5) and the patient and wherein the position of the reference star (5) relative to the patient is changed and the change in the position of the reference star (5) relative to the patient is detected by means of at least one position detector with a continuous display, which is attached to the bearing or joint (6), wherein the movement which the reference star (5) has performed with respect to its previous position can be continuously ascertained.

9. The method according to the preceding claim, wherein the detected change in the position of the reference star (5) is conveyed to a navigation system.

10. The method according to the preceding claim, wherein the structure of the reference star adaptor and/or the structure of the bearing or joint (6) is stored in a software of the navigation system, in a database.

11. A computer program which, when it is running on a computer or is loaded onto a computer, performs the method according to any one of the preceding three claims.

12. A program storage medium or a computer program product comprising the computer program according to the preceding claim.

## Revendications

1. Adaptateur pour étoile de référence comportant au moins un premier élément de fixation (8) afin de fixer l'adaptateur pour étoile de référence sur un patient, ainsi qu'au moins un deuxième élément de fixation (5) sur lequel peuvent être placés des repères (5a), au moins un palier ou articulation (6) étant prévu entre le premier élément de fixation (8) et le deuxième élément de fixation (5), **caractérisé en ce que** sur le au moins un palier ou articulation (6) est placé au moins un détecteur de position afin de déterminer dans quelle position se trouve le palier ou articulation (6).

2. Adaptateur pour étoile de référence selon la revendication 1, dans lequel le palier (6) est un palier libre ou un palier fixe, ou l'articulation est une articulation tournante, une articulation coulissante, une articulation à courbe, une articulation à vis, une articulation tournante et coulissante, une articulation sphérique ou une articulation à plaque.

3. Adaptateur pour étoile de référence selon l'une des revendications précédentes, dans lequel le détecteur de position peut mesurer un mouvement de translation.

4. Adaptateur pour étoile de référence selon l'une des revendications précédentes, dans lequel le détecteur de position peut mesurer un mouvement de rotation.

5. Adaptateur pour étoile de référence selon l'une des revendications précédentes, dans lequel le au moins un détecteur de position est relié à une unité de calcul (2).

6. Adaptateur pour étoile de référence selon l'une des revendications précédentes avec au moins un moteur qui peut modifier la position d'au moins un palier ou articulation (6).

7. Système de navigation avec un adaptateur pour étoile de référence selon l'une des revendications précédentes, une étoile de référence (5), des caméras (1) et une unité de calcul (2) reliée aux caméras (1).

8. Procédé pour un nouvel étalonnage ou nouveau repérage d'un patient avec une étoile de référence (5) fixée à celui-ci, dans lequel au moins un palier ou articulation (6) déplaçable en continu est prévu entre l'étoile de référence (5) et le patient et dans lequel la position de l'étoile de référence (5) est modifiée par rapport au patient et la modification de la position de l'étoile de référence (5) par rapport au patient est détectée au moyen d'au moins un détecteur de position avec un affichage en continu qui est placé sur le palier ou articulation (6), le mouvement que l'étoile de référence (5) exécute par rapport à sa position actuelle pouvant être déterminé de manière continue.

9. Procédé selon la revendication précédente, dans lequel la modification détectée de la position de l'étoile de référence (5) est transmise à un système de navigation.

10. Procédé selon la revendication précédente, dans lequel dans un logiciel du système de navigation, la structure de l'adaptateur pour étoile de référence et/ou du palier ou articulation (6) est mémorisée dans une banque de données.

11. Programme d'ordinateur qui met en oeuvre le procédé selon l'une des trois revendications précédentes, lorsqu'il tourne dans un ordinateur ou est chargé dans un ordinateur.

12. Support d'enregistrement de programme ou produit de programme d'ordinateur contenant le programme d'ordinateur selon la revendication précédente.
